# EUROPÄISCHE PATENTSCHRIFT

(11) **EP 0 508 348 B1**
(45) Veröffentlichungstag und Bekanntmachung des Hinweises auf die Patenterteilung: **09.07.1997**
(21) Anmeldenummer: 92105919.2
(22) Anmeldetag: 06.04.1992
(51) Int. Cl.: C07D 513/04, C07D 401/12

(54) **Verfahren zur Herstellung von 2-Pyridylsulfonylharnstoffen und Thiadiazolopyridine als Zwischenprodukte in diesem Verfahren**
Process for the preparation of 2-pyridylsulfonylureas and thiadiazolopyridines as intermediates in this process
Procédé pour la préparation de 2-pyridylsulfonylurées et thiadiazolopyridines comme intermédiaires dans ce procédé

(30) Priorität: 11.04.1991 DE 4111800
(43) Veröffentlichungstag der Anmeldung: 14.10.1992
(73) Patentinhaber: Hoechst Schering AgrEvo GmbH, 13342 Berlin (DE)
(72) Erfinder: Kehne, Heinz, Dr., W-6238 Hofheim am Taunus (DE)

(56) Entgegenhaltungen:
- EP-A- 0 013 480
- EP-A- 0 021 641
- EP-A- 0 184 385
- EP-A- 0 308 371
- EP-A- 0 317 336
- EP-A- 0 451 468

## Beschreibung

2-Pyridylsulfonylharnstoffe sind bekannte Herbizide mit vielfältigen Einsatzmöglichkeiten. Wichtige Vertreter dieser Substanzklasse sowie deren Herstellung und Verwendung sind beispielsweise bekannt aus EP-A-184385, WO 89/02700, WO 88/04297 oder DE-A-4000503 (ZA 91/0173).

Die daraus bekannten Herstellungsverfahren sind jedoch nicht optimal. Nachteilig erscheinen insbesondere die niedrigen Ausbeuten, die Verwendung von kostspieligen oder schlecht zu handhabenden Katalysatoren, wie z.B. 1,8-Diazabicyclo[5.4.0]undec-7-en (DBU) oder Trimethylaluminium, die begrenzte Anwendungsbreite der Reaktionen oder der Einsatz von Edukten oder Reagenzien in großem Überschuß.

Aufgabe der vorliegenden Erfindung ist es, unter Reduzierung oder Vermeidung der geschilderten Nachteile ein einfaches, ökonomisches und in industriellem Maßstab durchführbares Verfahren zur Verfügung zu stellen.

Gegenstand der vorliegenden Erfindung ist demgemäß ein Verfahren zur Herstellung von Verbindungen der Formel (I), 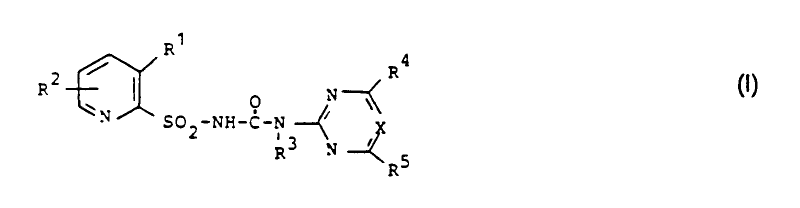 worin
- R¹: (C₁-C₄)Alkyl, das unsubstituiert oder durch ein oder mehrere Reste aus der Gruppe Halogen, Hydroxy, (C₁-C₄)Alkoxy, (C₁-C₄)Haloalkoxy und (C₂-C₈)Alkoxyalkoxy substituiert ist, -COR⁶, (C₁-C₄)Alkylsulfonyl, Halogen, NO₂, -ER⁷, -NR⁸R⁹ oder -SO₂NR¹⁰R¹¹,
- R²: H, (C₁-C₃)Alkyl, das unsubstituiert oder durch (C₁-C₃)Alkoxy, Hydroxy, (C₁-C₂)Alkylthio oder CN substituiert ist, (C₁-C₃)Haloalkyl, Halogen, CN, NO₂, (C₁-C₃)Alkoxy, (C₁-C₃)Alkylthio, (C₁-C₃)Haloalkoxy, (C₁-C₃)Alkylamino, Di(C₁-C₃)alkylamino oder (C₁-C₃)Alkylsulfonyl,
- R³: H oder CH₃,
- R⁴, R⁵: unabhängig voneinander (C₁-C₃)Alkyl, (C₁-C₃)Alkoxy, (C₁-C₃)Haloalkyl, (C₁-C₃)Haloalkoxy, (C₁-C₃)Alkylthio, Halogen, (C₂-C₅)Alkoxyalkyl oder (C₁-C₃)Alkylamino,
- X: CH oder N,
- R⁶: H, -NR¹⁰R¹¹, -ER¹² oder (C₁-C₄)Alkyl, das unsubstituiert oder durch ein oder mehrere Reste aus der Gruppe Halogen, (C₁-C₃)Alkoxy und (C₁-C₃)Alkylthio substituiert ist,
- E: ein Sauerstoff- oder Schwefelatom,
- R⁷: (C₁-C₄)Alkyl, das unsubstituiert oder durch ein oder mehrere Reste aus der Gruppe Halogen, (C₁-C₃)Alkoxy, (C₃-C₄)Alkenyloxy, (C₃-C₄)Alkinyloxy, (C₁-C₃)Alkylthio und [(C₁-C₄)-Alkoxy]-carbonyl substituiert ist, (C₃-C₄)Alkenyl oder (C₃-C₄)Alkinyl,
- R⁸: (C₁-C₄)Alkyl, (C₁-C₄)Haloalkyl oder (C₂-C₆)Alkoxyalkyl,
- R⁹: (C₁-C₄)Alkylsulfonyl, das unsubstituiert oder durch ein oder mehrere Reste aus der Gruppe Halogen und (C₁-C₄)Alkoxy substituiert ist,
- R¹⁰, R¹¹: unabhängig voneinander H, (C₁-C₃)Alkyl, (C₁-C₃)Haloalkyl, (C₁-C₂)Alkoxy, (C₃-C₄)Alkenyl oder (C₃-C₄)Alkinyl oder
- R¹⁰ und R¹¹: gemeinsam eine Alkylenbrücke der Formel -(CH₂)ₙ-, worin n = 2, 3, 4 oder 5 bedeutet, wobei die Alkylenbrücke durch ein Sauerstoffatom unterbrochen sein kann, und
- R¹²: (C₁-C₄)Alkyl, das unsubstituiert oder durch ein oder mehrere Reste aus der Gruppe Halogen, (C₁-C₃)Alkoxy, (C₃-C₄)Alkenyloxy, (C₃-C₄)Alkinyloxy, Phenoxy, (C₁-C₃)Alkylthio oder [(C₁-C₄)Alkoxy]-carbonyl substituiert ist, (C₃-C₄)Alkenyl, (C₃-C₄)Alkinyl oder Phenyl
bedeuten,
dadurch gekennzeichnet, daß man ein zwitterionisches 1,1,3-Trioxo-1,2,4-thiadiazolo[4,5-a]pyridin der Formel (II), 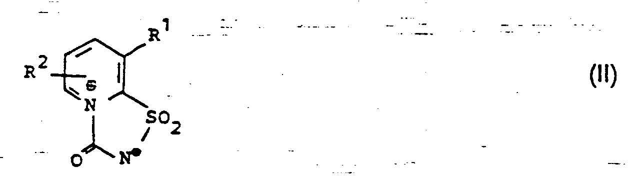 worin R¹ und R² dieselbe Bedeutung wie in Formel (I) haben,
mit einem Aminoheterocyclus der Formel (III) 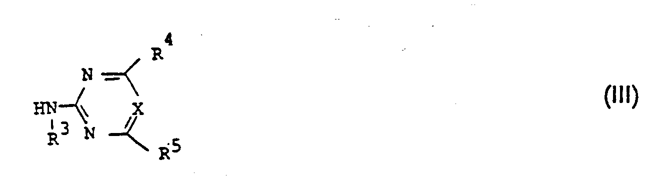 worin R³, R^{4,} R⁵ und X dieselbe Bedeutung wie in Formel (I) haben, in einem inerten organischen Lösungsmittel umsetzt.

In den obengenannten Formeln können Alkyl-, Alkoxy-, Haloalkyl-, Alkylamino- und Alkylthioreste sowie die entsprechenden ungesättigten und/oder substituierten Reste, wenn nicht anders angegeben, jeweils geradkettig oder verzweigt sein. Alkylreste, auch in den zusammengesetzten Bedeutungen wie Alkoxy, Haloalkyl, Alkoxycarbonyl, Alkoxyalkoxy usw., bedeuten beispielsweise Methyl, Ethyl, n- oder i-Propyl, n-, i-, t- oder 2-Butyl; Alkenyl- und Alkinylreste haben die Bedeutung der den Alkylresten entsprechenden möglichen ungesättigten Reste, wie 2-Propenyl, 2- oder 3-Butenyl, 2-Propinyl, 2- oder 3-Butinyl, Halogen bedeutet Fluor, Chlor, Brom oder Iod. Haloalkyl oder Haloalkoxy bedeuten Alkyl bzw. Alkoxy, die durch ein oder mehrere Reste aus der Gruppe F, Cl, Br und Iod, vorzugsweise Fluor oder Chlor substituiert sind.

Von besonderem Interesse ist das erfindungsgemäße Verfahren, worin
- R¹: (C₁-C₄)Alkyl, das unsubstituiert oder durch ein oder mehrere Halogenatome oder ein oder zwei, vorzugsweise ein Hydroxy oder (C₁-C₄)Alkoxy oder durch ein oder mehrere Halogenatome und ein oder zwei, vorzugsweise ein Hydroxy oder (C₁-C₄)Alkoxy substituiert ist, -COR⁶, (C₁-C₄)Alkylsulfonyl, Halogen oder -NR⁸R⁹,
- R²: H, (C₁-C₃)Alkyl, das unsubstituiert oder durch (C₁-C₃)Alkoxy, Hydroxy, (C₁-C₂)Alkylthio oder Cyano substituiert ist, (C₁-C₃)Alkylthio, (C₁-C₃)Haloalkoxy, (C₁-C₃)Alkylamino oder Di(C₁-C₃)alkylamino,
- R³: H oder CH₃,
- R⁴, R⁵: unabhängig voneinander (C₁-C₂)Alkyl, (C₁-C₃)Alkoxy, (C₁-C₂)Haloalkyl, (C₁-C₂)Haloalkoxy, Halogen oder (C₁-C₂)Alkylamino,
- X: CH oder N,
- R⁶: H, -ER¹² oder (C₁-C₄)Alkyl,
- E: ein Sauerstoffatom,
- R⁸: (C₁-C₄)Alkyl oder (C₁-C₄)Haloalkyl,
- R⁹: (C₁-C₄)Alkylsulfonyl und
- R¹²: (C₁-C₄)Alkyl
bedeuten.

Die Umsetzung der Verbindungen der Formeln II und III wird beispielsweise in einem unter den Reaktionsbedingungen inerten organischen Lösungsmittel bei Temperaturen von -10 °C bis zur Siedetemperatur des Lösungsmittels durchgeführt, bevorzugt bei 0 °C bis 80 °C. Als Lösungsmittel eignen sich beispielsweise halogenierte aliphatische oder aromatische Kohlenwasserstoffe wie Dichlormethan oder Chlorbenzol, aromatische Kohlenwasserstoffe, wie Toluol oder Xylol, Ether wie Diethylether, Dimethoxyethan, Diethylenglykoldimethylether, Dioxan oder Tetrahydrofuran, Ester wie Ethylacetat oder Butylacetat, Ketone wie Aceton oder Methylisobutylketon, Nitrile wie Acetonitril oder Amide wie Dimethylformamid.

Die Edukte der Formeln (II) und (III) werden dabei zweckmäßig in dem jeweiligen Lösungsmittel separat gelöst bzw. suspendiert, gegebenenfalls abgekühlt und zusammengegeben. Danach wird das Reaktionsgemisch bei der jeweiligen Reaktionstemperatur solange gerührt, bis die Umsetzung beendet ist. Das Reaktionsprodukt der Formel (I) kann dann häufig einfach abgesaugt werden.

Alternativ können andere übliche Methoden zur Aufarbeitung und Isolierung des Produkts angewendet werden. Beispielsweise kann man in der Regel das Lösungsmittel destillativ entfernen und das anfallende Rohprodukt, falls erforderlich, mit geeigneten Methoden weiter reinigen.

Während die Aminoheterocyclen der Formel (III) literaturbekannt sind (vgl. "The Chemistry of Heterocyclic Compounds" Bd. XVI (1962) und Supplement I sowie Bd. XIII (1959), Interscience Publ. New York & London), handelt es sich bei den zwitterionischen Thiadiazolopyridinen der Formel (II) um neue Verbindungen. Gegenstand der vorliegenden Erfindung sind demgemäß auch die 1,1,3-Trioxo-1,2,4-thiadiazolo[4,5-a]pyridine der Formel (II) und deren Herstellung. Die Verbindungen der Formel (II) erhält man beispielsweise ausgehend von N-Pyridylsulfonyl-N'-alkylharnstoffen der Formel (IV), 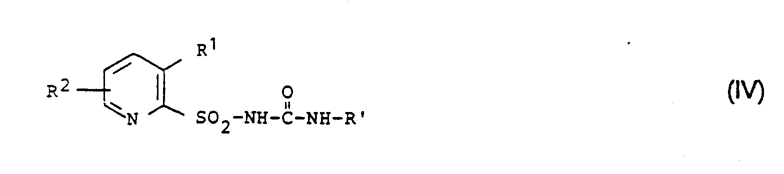 worin R¹ und R² wie in der genannten Formel (I) definiert sind und R' (C₁-C₈)Alkyl bedeutet, durch Umsetzung mit Phosgen. Man führt die Reaktion in unter den Reaktionsbedingungen inerten organischen Lösungsmitteln wie beispielsweise aromatischen Kohlenwasserstoffen, z.B. Toluol, Xylol oder Tetralin, oder halogenierten aromatischen Kohlenwasserstoffen, z.B. Chlorbenzol oder Dichlorbenzol, bei Temperaturen zwischen 70 °C und dem Siedepunkt des Lösungsmittels, insbesondere 70 °C bis 140 °C, durch. Dabei verwendet man pro Mol Alkylsulfonylharnstoff der Formel (IV) vorzugsweise 1,0-10,0 Mol Phosgen. Zur Beschleunigung der Reaktion kann gegebenenfalls eine nicht wäßrige Base, z.B. eine organische Aminbase, wie ein tertiäres Amin, z.B. Triethylamin oder 1,4-Diazabicyclo[2.2.2]octan (DABCO), als Katalysator zugesetzt werden. Das bei der Reaktion gebildete Alkylisocyanat kann man destillativ aus dem Reaktionsgemisch zurückgewinnen, während das Produkt (II) üblicherweise nach Abkühlen aus der Lösung ausfällt und durch Absaugen isolierbar ist. Die Ausbeuten bei diesem Prozeß liegen im allgemeinen in der Größenordnung von 90 % d. Th..

Die Ausgangsmaterialien der Formel (IV) für die Phosgenierungsreaktion sind literaturbekannt oder lassen sich nach literaturbekannten Verfahren herstellen (vgl. Boggiano et al., J. of Pharmacy and Pharmacology 13, 567 (1961), WO 87/07114 oder EP-A-336354).

Die Herstellung von Verbindungen der Formel (I) aus Verbindungen der Formel (IV) kann beispielsweise als Zweistufenverfahren mit Isolierung des zwitterionischen Thiadiazolopyridins der Formel (II) und dessen anschließender Umsetzung zum Sulfonylharnstoff der Formel (I) durchgeführt werden oder man verzichtet auf die Isolierung von der Verbindung der Formel (II) und setzt stattdessen zum Sulfonylharnstoff der Formel (I) um, indem man nach Bildung von (II) den Aminoheterocyclus (III) direkt zum Reaktionsgemisch der ersten Stufe gibt (Eintopfreaktion).

Des weiteren ist es möglich, ausgehend von einem Sulfonamid der Formel (V), 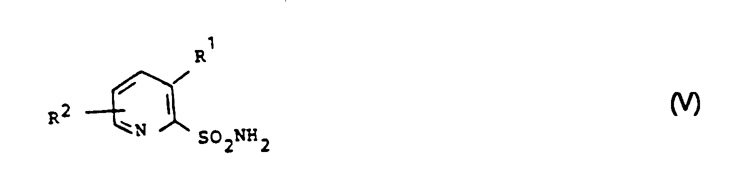 worin R¹ und R² wie in Formel (I) definiert sind, direkt in Gegenwart von einem (C₁-C₈)Alkylisocyanat und gegebenenfalls einer der genannten nicht wäßrigen Basen, z.B. einem tertiären Amin wie Triethylamin oder DABCO, als Katalysator zu phosgenieren. In diesem Fall werden die Alkylsulfonylharnstoffe der Formel (IV) nicht isoliert, sondern nur in situ gebildet und sofort weiter zu Verbindungen der Formel (II) umgesetzt. Die Verbindungen der Formel (V)sind bekannt (s. z.B. WO 88/04297, DE-A-4 000 503 (ZA 91/0173)).

Das erfindungsgemäße Verfahren soll durch die folgenden Beispiele näher erläutert werden:

### Beispiel 1

### 7-(N-Methyl-N-methylsulfonyl-amino)-1,1,3-trioxo-1,2,4-thiadiazolo[4,5-a]pyridin der Formel (II-1):

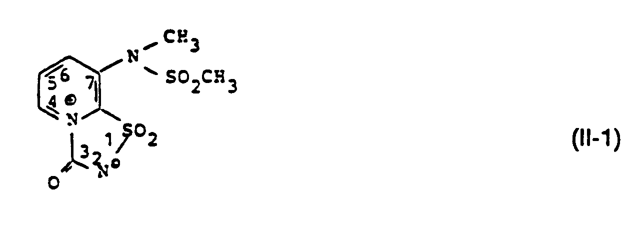

36,5 g (0,1 mol) 3-Butyl-1-[3-(N-methyl-N-methylsulfonyl-amino)-2-pyridylsulfonyl]-harnstoff werden in 600 ml Chlorbenzol suspendiert und auf 70 °C erhitzt. Man beginnt bei dieser Temperatur, einen gelinden Phosgenstrom in die Suspension einzuleiten und heizt ca. 20 min kontinuierlich weiter bis eine Innentemperatur von 120 °C erreicht ist. Nach weiteren 20 Minuten mit Einleiten von Phosgen (insgesamt ca. 30 g (0,3 mol) Phosgen) werden die niedrigsiedenden Anteile zusammen mit ca. 30 % des Lösungsmittels innerhalb von 20 min aus dem Reaktionsgemisch abdestilliert. Nach Abkühlen auf Raumtemperatur, Filtrieren und Waschen des Feststoffs mit Diethylether erhält man 26,7 g (92 % d. Th.) der Verbindung der Formel (II-1) vom Schmp. 208-209 °C (Zers.).
¹H-NMR (80 MHz, d₆-DMSO):
- δ [ppm] =: 8,40 (dd, J¹ = 9Hz, J² = 7Hz; 1H, 5-H)
8,95 (d, J = 9Hz; 1H, 6-H)
9,35 (d, H = 7Hz; 1H, 4-H)
- IR (KBr): ν =: 1810 cm⁻¹ (C = O), kein Signal zwischen 2200 und 2300 cm⁻¹.

In ähnlicher Weise wie in Beispiel 1 erhält man auch die in Tabelle 1 aufgeführten Verbindungen der Formel II.

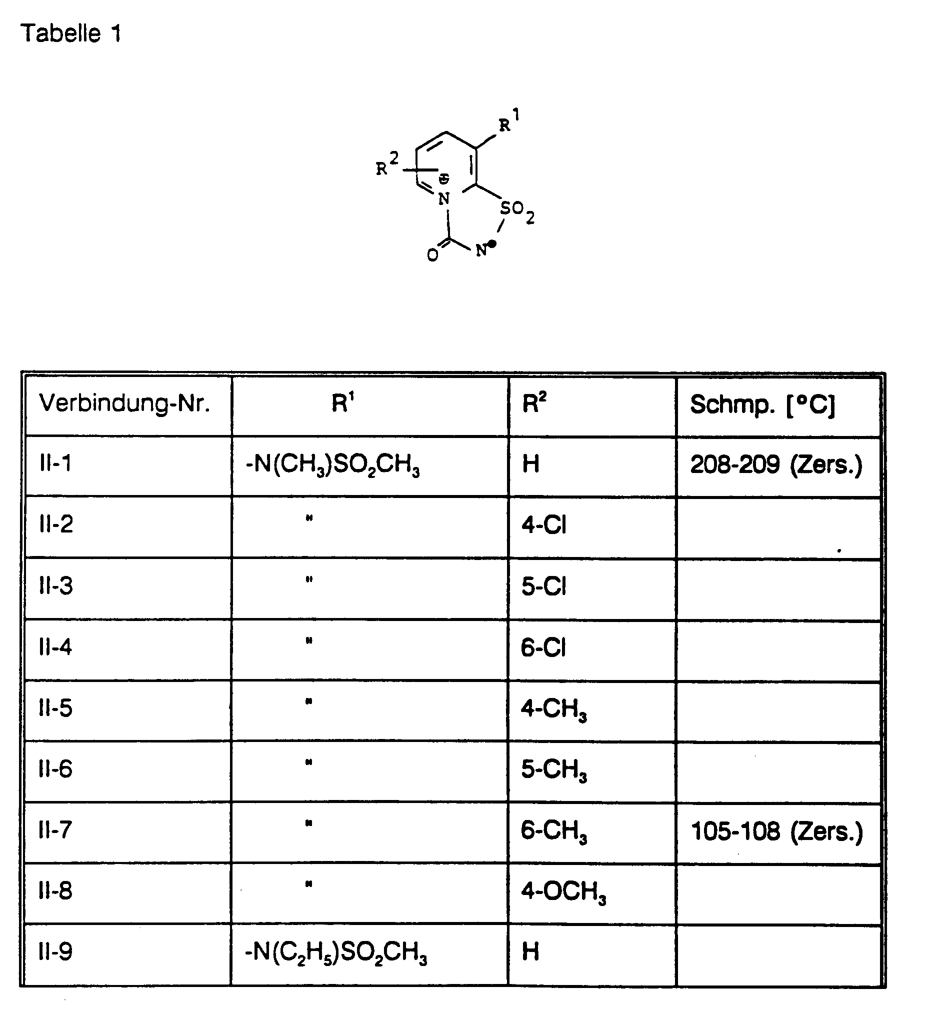

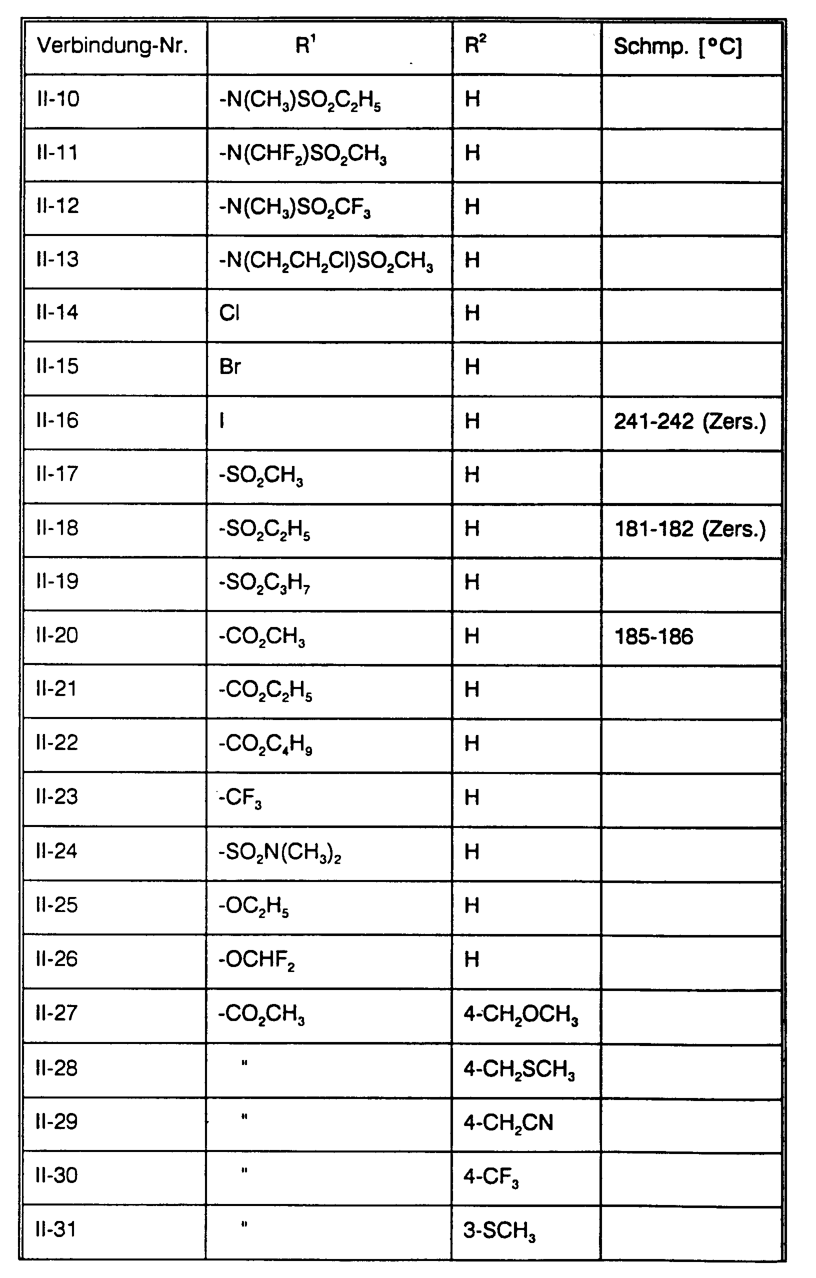

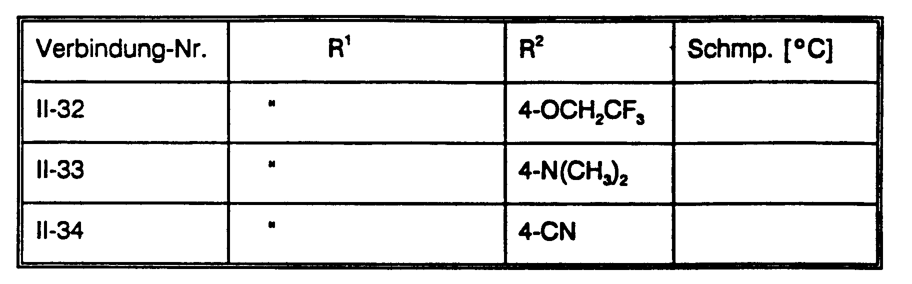

### Beispiel 2

### 7-(N-Methyl-N-methylsulfonyl-amino)-1,1,3-trioxo-1,2,4-thiadiazolo[4,5-a]pyridin der Formel (II-1) ausgehend vom entsprechenden Sulfonamid:

Eine Lösung von 26,5 g (0,1 mol) 3-(N-Methyl-N-methylsulfonylamino)-2-pyridylsulfonamid, 10,0 g (0,1 mol) Butylisocyanat und 0,1 g 1,4-Diazabicyclo[2.2.2]octan in 150 ml Chlorbenzol wird 20 min zum Rückfluß erhitzt. Anschließend leitet man ebenfalls bei Rückflußtemperatur für 1 h Phosgen ein. Nachdem man die niedrigsiedenden Anteile unter Normaldurck abdestilliert hat, kühlt man auf Raumtemperatur und filtriert den Feststoff ab. Nach Waschen mit Diethylether erhält man 25,0 g (86 % d. Th.) der Verbindung (II-1) mit denselben physikalischen Eigenschaften wie in Beispiel 1 beschrieben.

### Beispiel 3

### 3-(4-Methoxy-6-methyl-1,3,5-triazin-2-yl)-3-methyl-1-[3-(N-methyl-N-methylsulfonylamino)-2-pyridylsulfonyl]-harnstoff

2,9 g (10 mmol) der Verbindung (II-1) (Beispiel 1) und 1,6 g (10,5 mmol) 4-Methoxy-6-methyl-2-methylamino-1,3,5-triazin werden in 100 ml Dichlormethan zusammengegeben und 24 h zum Rückfluß erwärmt. Anschließend kühlt man ab, wäscht die organische Phase 1 x mit je 50 ml 1 N HCl und Wasser, trocknet und dampft zur Trockne ein. Nach Verreiben des Rohprodukts mit Diethylether hinterbleiben 3,9 g (88 % d. Th.) 3-(4-Methoxy-6-methyl-1,3,5-triazin-2-yl)-3-methyl-1-[3-(N-methyl-N-methylsulfonyl-amino)-2-pyridylsulfonyl]-harnstoff vom Schmp. 142-144 °C (Zers.).

### Beispiel 4

### 3-(4,6-Dimethoxypyrimidin-2-yl)-1-[3-(N-methyl-N-methylsulfonyl-amino)-2-pyridylsulfonyl]-harnstoff

2,9 g (10 mmol) der Verbindung (II-1) (Beispiel 1) und 1,55 g (10 mmol) 2-Amino-4,6-dimethoxypyrimidin werden in 30 ml Dichlormethan zusammengegeben und 8 h zum Rückfluß erwärmt. Nach Entfernen des Lösungsmittels im Vakuum erhält man 4,45 g (100 % d. Th.) 3-(4,6-Dimethoxypyrimidin-2-yl)-1-[3-(N-methyl-N-methylsulfonylamino)-2-pyridylsulfonyl]-harnstoff vom Schmp. 168-173 °C (Zers.) in einer Reinheit von ca. 90 %.

### Beispiel 5

### 3-(4,6-Dimethoxypyrimidin-2-yl)-3-methyl-1-[3-(N-methyl-N-methylsulfonyl-amino)-2-pyridylsulfonyl]-harnstoff (Eintopfreaktion)

63,0 g (0,17 mol) 3-Butyl-1-[3-(N-methyl-N-methylsulfonylamino)-2-pyridylsulfonyl]-harnstoff werden in 1 l Chlorbenzol suspendiert und auf 70 °C erhitzt. Man beginnt bei dieser Temperatur, einen gelinden Phosgenstrom in die Suspension einzuleiten und heizt ca. 20 min kontinuierlich weiter bis eine Innentemperatur von 120 °C erreicht ist. Nach weiterem 20 minütigem Einleiten von Phosgen (insgesamt ca. 50 g (0,5 mol)) werden die niedrigsiedenden Anteile zusammen mit ca. 300 ml Chlorbenzol innerhalb von 30 min aus dem Reaktionsgemisch abdestilliert. Nach Abkühlen auf Raumtemperatur gibt man 29,0 g (0,17 mol) 4,6-Dimethoxy-2-methylaminopyrimidin gelöst in 500 ml Dichlormethan zu und erwärmt 7 h auf 40 °C. Man kühlt ab, wäscht die organische Phase mit je 1 x 250 ml 1 N HCl und Wasser, trocknet und dampft ein. Nach Verreiben des Rohprodukts mit Diethylether hinterbleiben 66,6 g (85 % d. Th.) 3-(4,6-Dimethoxypyrimidin-2-yl)-3-methyl-1-[3-(N-methyl-N-methylsulfonyl-amino)-2-pyridylsulfonyl]-harnstoff vom Schmp. 167-168 °C (Zers.).

Die weiteren Verbindungen der allgemeinen Formel (I) können in analoger Weise erhalten werden.

## Patentansprüche

1. Verfahren zur Herstellung von Verbindungen der Formel (I), 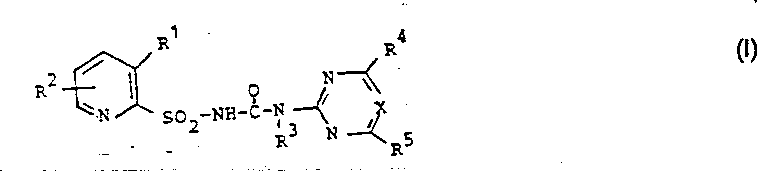 worin
R¹ (C₁-C₄)Alkyl, das unsubstituiert oder durch ein oder mehrere Reste aus der Gruppe Halogen, Hydroxy, (C₁-C₄)Alkoxy, (C₁-C₄)Haloalkoxy und (C₂-C₈)Alkoxyalkoxy substituiert ist, -COR⁶, (C₁-C₄)Alkylsulfonyl, Halogen, NO₂, -ER⁷, -NR⁸R⁹ oder -SO₂NR¹°R¹¹,
R² H, (C₁-C₃)Alkyl, das unsubstituiert oder durch (C₁-C₃)Alkoxy, Hydroxy, (C₁-C₂)Alkylthio oder CN substituiert ist, (C₁-C₃)Haloalkyl, Halogen, CN, NO₂, (C₁-C₃)Alkoxy, (C₁-C₃)Alkylthio, (C₁-C₃)Haloalkoxy, (C₁-C₃)Alkylamino, Di(C₁-C₃)alkylamino oder (C₁-C₃)Alkylsulfonyl,
R³ H oder CH₃,
R⁴, R⁵ unabhängig voneinander (C₁-C₃)Alkyl, (C₁-C₃)Alkoxy, (C₁-C₃)Haloalkyl, (C₁-C₃)Haloalkoxy, (C₁-C₃)Alkylthio, Halogen, (C₂-C₅)Alkoxyalkyl oder (C₁-C₃)Alkylamino,
X CH oder N,
R⁶ H, -NR¹⁰R¹¹, -ER¹² oder (C₁-C₄)Alkyl, das unsubstituiert oder durch ein oder mehrere Reste aus der Gruppe Halogen, (C₁-C₃)Alkoxy und (C₁-C₃)Alkylthio substituiert ist,
E ein Sauerstoff- oder Schwefelatom,
R⁷ (C₁-C₄)Alkyl, das unsubstituiert oder durch ein oder mehrere Reste aus der Gruppe Halogen, (C₁-C₃)Alkoxy, (C₃-C₄)Alkenyloxy, (C₃-C₄)Alkinyloxy, (C₁-C₃)Alkylthio und [(C₁-C₄)Alkoxy]-carbonyl substituiert ist, (C₃-C₄)Alkenyl oder (C₃-C₄)Alkinyl,
R⁸ (C₁-C₄)Alkyl, (C₁-C₄)Haloalkyl oder (C₂-C₆)Alkoxyalkyl,
R⁹ (C₁-C₄)Alkylsulfonyl, das unsubstituiert oder durch ein oder mehrere Reste aus der Gruppe Halogen und (C₁-C₄)Alkoxy substituiert ist,
R¹⁰, R¹¹ unabhängig voneinander H, (C₁-C₃)Alkyl, (C₁-C₃)Haloalkyl, (C₁-C₂)Alkoxy, (C₃-C₄)Alkenyl oder (C₃-C₄)Alkinyl oder
R¹⁰ und R¹¹ gemeinsam eine Alkylenbrücke der Formel -(CH₂)ₙ-, worin n = 2, 3, 4 oder 5 bedeutet, wobei die Alkylenbrücke durch ein Sauerstoffatom unterbrochen sein kann, und
R¹² (C₁-C₄)Alkyl, das unsubstituiert oder durch ein oder mehrere Reste aus der Gruppe Halogen, (C₁-C₃)Alkoxy, (C₃-C₄)Alkenyloxy, (C₃-C₄)Alkinyloxy, Phenoxy, (C₁-C₃)Alkylthio oder [(C₁-C₄)Alkoxy]-carbonyl substituiert ist, (C₃-C₄)Alkenyl, (C₃-C₄)Alkinyl oder Phenyl
bedeuten,
dadurch gekennzeichnet, daß man ein zwitterionisches 1,1,3-Trioxo-1,2,4-thiadiazolo[4,5-a]pyridin der Formel (II), 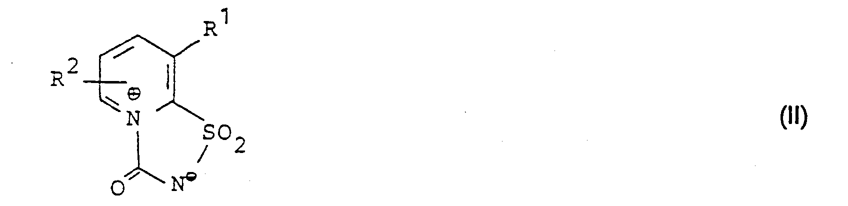 worin R¹ und R² dieselbe Bedeutung wie in Formel (I) haben,
mit einem Aminoheterocyclus der Formel (III) 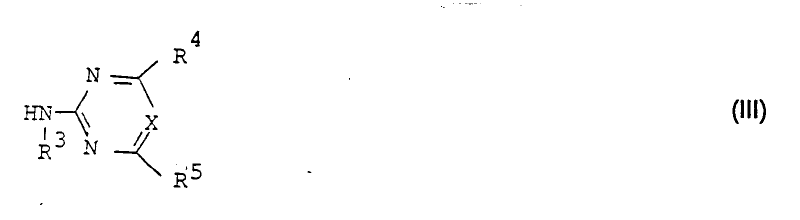 worin R³, R⁴, R⁵ und X dieselbe Bedeutung wie in Formel (I) haben, in einem inerten organischen Lösungsmittel umsetzt.

2. Verfahren nach Anspruch 1, dadurch gekennzeichnet, daß
R¹ (C₁-C₄)Alkyl, das unsubstituiert oder durch ein oder mehrere Halogenatome oder ein oder zwei Hydroxy oder (C₁-C₄)Alkoxy oder durch ein oder mehrere Halogenatome und ein oder zwei Hydroxy oder (C₁-C₄)Alkoxy substituiert ist, -COR⁶, (C₁-C₄)Alkylsulfonyl, Halogen oder -NR⁸R⁹
R² H, (C₁-C₃)Alkyl, das unsubstituiert oder durch (C₁-C₃)Alkoxy, Hydroxy, (C₁-C₂)Alkylthio oder -CN substituiert ist, (C₁-C₃)Alkylthio, (C₁-C₃)Haloalkoxy, (C₁-C₃)Alkylamino oder Di(C₁-C₃)alkylamino,
R³ H oder CH₃,
R⁴, R⁵ unabhängig voneinander (C₁-C₂)Alkyl, (C₁-C₃)Alkoxy, (C₁-C₂)Haloalkyl, (C₁-C₂)Haloalkoxy, Halogen oder (C₁-C₂)Alkylamino,
X CH oder N,
R⁶ H, -ER¹² oder (C₁-C₄)Alkyl,
E ein Sauerstoffatom,
R⁸ (C₁-C₄)Alkyl oder (C₁-C₄)Haloalkyl,
R⁹ (C₁-C₄)Alkylsulfonyl und
R¹² (C₁-C₄)Alkyl
bedeuten.

3. Verfahren nach Anspruch 1 oder 2, dadurch gekennzeichnet, daß ein inertes organisches Lösungsmittel aus der Gruppe aromatische Kohlenwasserstoffe, halogenierte aliphatische und aromatische Kohlenwasserstoffe, Ether, Ester, Ketone, Nitrile und Amide sowie Gemische davon eingesetzt wird.

4. Verfahren nach Anspruch 3, dadurch gekennzeichnet, daß Chlorbenzol oder Dichlormethan als inertes organisches Lösungsmittel eingesetzt wird.

5. Verfahren nach Anspruch 3 oder 4, dadurch gekennzeichnet, daß man die Umsetzung bei Temperaturen von -10 °C bis zur Siedetemperatur des Lösungsmittels durchführt.

6. Verfahren nach Anspruch 5, dadurch gekennzeichnet, daß die Temperaturen von 0 °C bis 80 °C sind.

7. Verbindungen der Formel II, 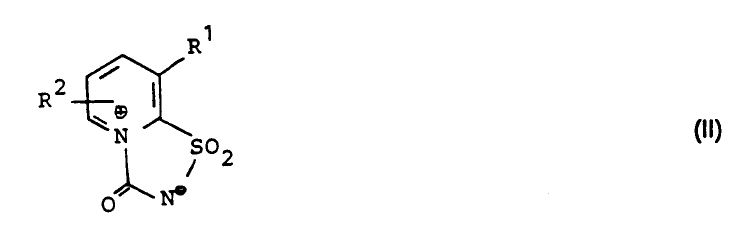 worin R¹ und R² wie in Formel (I) nach Anspruch 1 oder 2 definiert sind.

8. Verfahren zur Herstellung von Verbindungen der Formel II nach Anspruch 7, dadurch gekennzeichnet, daß man Verbindungen der Formel IV, 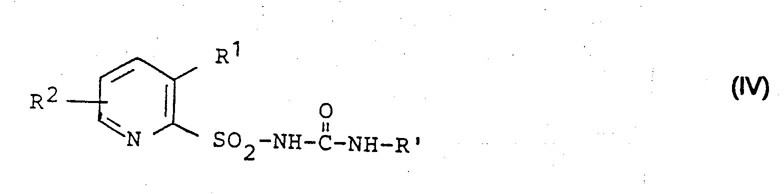 worin
R¹ und R² wie in Formel II definiert sind und R' (C₁-C₈)Alkyl bedeutet, in Gegenwart eines inerten organischen Lösungsmittels bei Temperaturen zwischen 70 °C und dem Siedepunkt des Lösungsmittels mit Phosgen umsetzt.

9. Verfahren nach Anspruch 8, dadurch gekennzeichnet, daß man die Umsetzung bei Temperaturen von 70 °C bis 140 °C durchführt.

10. Verfahren zur Herstellung von Verbindungen der Formel II nach Anspruch 7, dadurch gekennzeichnet, daß man eine Verbindung der Formel V, 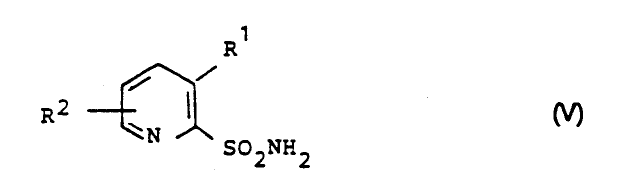 worin R¹ und R² wie in Formel II definiert sind, in Gegenwart eines (C₁-C₈)-Alkylisocyanats und Phosgen umsetzt.

11. Verfahren nach Anspruch 10, dadurch gekennzeichnet, daß die Umsetzung in einem inerten organischen Lösungsmittel bei Temperaturen zwischen 70 °C und dem Siedepunkt des Lösungsmittels durchgeführt wird.

12. Verfahren nach Anspruch 8 oder 9 oder 10 oder 11, dadurch gekennzeichnet, daß man zur Beschleunigung der Umsetzung einen Katalysator aus der Gruppe der tertiären Amine einsetzt.

13. Verwendung von Verbindungen der Formel II nach Anspruch 7 zur Herstellung von Sulfonylharnstoffen.

## Claims

1. A process for the preparation of compounds of the formula (I) 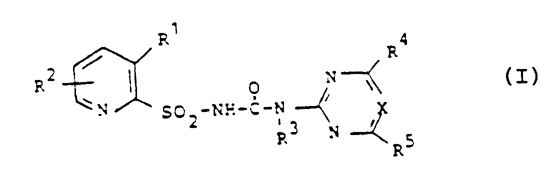 in which
R¹ is (C₁-C₄)alkyl, which is unsubstituted or substituted by one or more radicals from the group comprising halogen, hydroxyl, (C₁-C₄)alkoxy, (C₁-C₄)haloalkoxy and (C₂-C₈)alkoxyalkoxy, or is -COR⁶, (C₁-C₄)alkylsulfonyl, halogen, NO₂, -ER⁷, -NR⁸R⁹ or -SO₂NR¹⁰R¹¹,
R² is H, (C₁-C₃) alkyl which is unsubstituted or substituted by (C₁-C₃)alkoxy, hydroxyl, (C₁-C₂)alkylthio or CN, or is (C₁-C₃)haloalkyl, halogen, CN, NO₂, (C₁-C₃)alkoxy, (C₁-C₃)alkylthio, (C₁-C₃)haloalkoxy, (C₁-C₃)alkylamino, di(C₁-C₃)alkylamino or (C₁-C₃)alkylsulfonyl,
R³ is H or CH₃,
R⁴ and R⁵ independently of one another are (C₁-C₃)alkyl, (C₁-C₃)alkoxy, (C₁-C₃)haloalkyl, (C₁-C₃)haloalkoxy, (C₁-C₃)alkylthio, halogen, (C₂-C₅)alkoxyalkyl or (C₁-C₃)alkylamino,
X is CH or N,
R⁶ is H, -NR¹⁰R¹¹, -ER¹² or (C₁-C₄)alkyl which is unsubstituted or substituted by one or more radicals from the group comprising halogen, (C₁-C₃)alkoxy and (C₁-C₃)alkylthio,
E is an oxygen or sulfur atom,
R⁷ is (C₁-C₄)alkyl which is unsubstituted or substituted by one or more radicals from the group comprising halogen, (C₁-C₃)alkoxy, (C₃-C₄)alkenyloxy, (C₃-C₄)alkynyloxy, (C₁-C₃)alkylthio and [(C₁-C₄)alkoxy]carbonyl, or is (C₃-C₄)alkenyl or (C₃-C₄)alkynyl,
R⁸ is (C₁-C₄)alkyl, (C₁-C₄)haloalkyl or (C₂-C₆)alkoxyalkyl,
R⁹ is (C₁-C₄)alkylsulfonyl which is unsubstituted or substituted by one or more radicals from the group comprising halogen and (C₁-C₄)alkoxy,
R¹⁰ and R¹¹ independently of one another are H, (C₁-C₃)alkyl, (C₁-C₃)haloalkyl, (C₁-C₂)alkoxy, (C₃-C₄)alkenyl or (C₃-C₄)alkynyl, or
R¹⁰ and R¹¹ together are an alkylene bridge of the formula -(CH₂)ₙ-, in which n is 2, 3, 4 or 5, it being possible for the alkylene bridge to be interrupted by an oxygen atom, and
R¹² is (C₁-C₄)alkyl which is unsubstituted or substituted by one or more radicals from the group comprising halogen, (C₁-C₃)alkoxy, (C₃-C₄)alkenyloxy, (C₃-C₄)alkynyloxy, phenoxy, (C₁-C₃)alkylthio or [(C₁-C₄)alkoxy]carbonyl, or is (C₃-C₄)alkenyl, (C₃-C₄)alkynyl or phenyl,
which comprises reacting a zwitterionic 1,1,3-trioxo-1,2,4-thiadiazolo[4,5-a]pyridine of the formula (II) 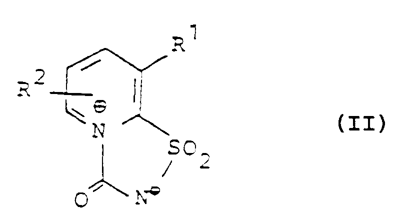 in which R¹ and R² have the same meaning as in formula (I),
with an aminoheterocycle of the formula (III) 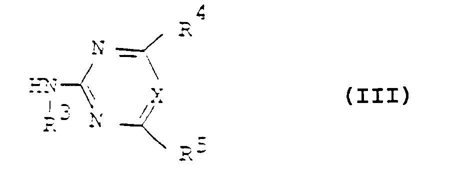 in which R³, R⁴, R⁵ and X have the same meaning as in formula (I), in an inert organic solvent.

2. The process as claimed in claim 1, wherein in the formula (I)
R¹ is (C₁-C₄)alkyl, which is unsubstituted or substituted by one or more halogen atoms or one or two hydroxyls or (C₁-C₄)alkoxy or by one or more halogen atoms and one or two hydroxyls or (C₁-C₄)alkoxy, or is -COR⁶, (C₁-C₄)alkylsulfonyl, halogen or -NR⁸R⁹,
R² is H, (C₁-C₃)alkyl which is unsubstituted or substituted by (C₁-C₃)alkoxy, hydroxyl, (C₁-C₂)alkylthio or -CN, or is (C₁-C₃)alkylthio, (C₁-C₃)haloalkoxy, (C₁-C₃)alkylamino or di(C₁-C₃)alkylamino,
R³ is H or CH₃,
R⁴ and R⁵ independently of one another are (C₁-C₂)alkyl, (C₁-C₃)alkoxy, (C₁-C₂)haloalkyl, (C₁-C₂)haloalkoxy, halogen or (C₁-C₂)alkylamino,
X is CH or N,
R⁶ is H, -ER¹² or (C₁-C₄)alkyl,
E is an oxygen atom,
R⁸ is (C₁-C₄)alkyl or (C₁-C₄)haloalkyl,
R⁹ is (C₁-C₄)alkylsulfonyl and
R¹² is (C₁-C₄) alkyl.

3. The process as claimed in claim 1 or 2, wherein an inert organic solvent from the group consisting of aromatic hydrocarbons, halogenated aliphatic and aromatic hydrocarbons, ethers, esters, ketones, nitriles and amides, and mixtures of these, is employed.

4. The process as claimed in claim 3, wherein the inert organic solvent employed is chlorobenzene or dichloromethane.

5. The process as claimed in claim 3 or 4, wherein the reaction is carried out at temperatures from -10°C up to the boiling point of the solvent.

6. The process as claimed in claim 5, wherein the temperatures are from 0°C to 80°C.

7. A compound of the formula II 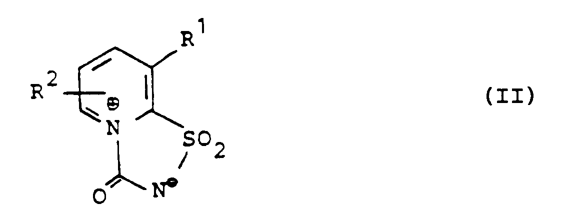 in which R¹ and R² are as defined in claim 1 or 2.

8. A process for the preparation of compounds of the formula II as claimed in claim 7, which comprises reacting compounds of the formula IV 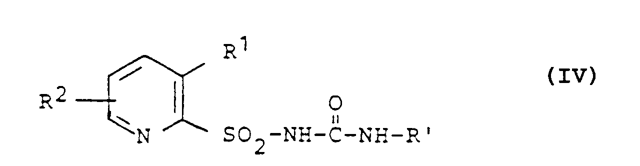 in which
R¹ and R² are as defined in formula II and R' is (C₁-C₈)alkyl, with phosgene in the presence of an inert organic solvent at temperatures between 70°C and the boiling point of the solvent.

9. The process as claimed in claim 8, wherein the reaction is carried out at temperatures from 70°C to 140°C.

10. A process for the preparation of compounds of the formula II as claimed in claim 7, which comprises reacting a compound of the formula V 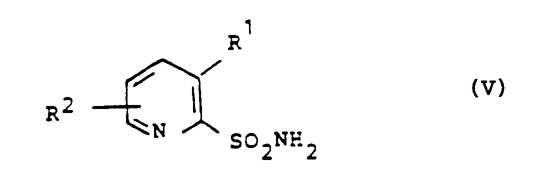 in which R¹ and R² are as defined in formula II, in the presence of a (C₁-C₈)alkyl isocyanate and phosgene.

11. The process as claimed in claim 10, wherein the reaction is carried out in an inert organic solvent at temperatures between 70°C and the boiling point of the solvent.

12. The process as claimed in claim 8 or 9 or 10 or 11, wherein a catalyst from the group of the tertiary amines is employed to accelerate the reaction.

13. The use of compounds of the formula II as claimed in claim 7 for the preparation of sulfonylureas.

## Revendications

1. Procédé de préparation de composés de formule (I) 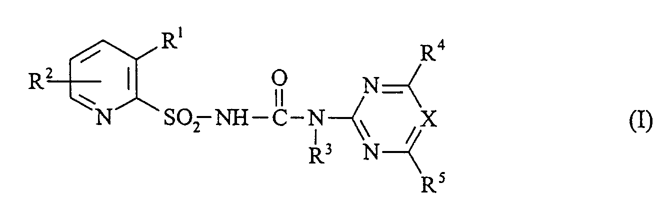 dans laquelle
R¹ représente un reste alkyle en C₁-C₄ non substitué ou substitué par un ou plusieurs restes du groupe constitué par des atomes d'halogène et des restes hydroxy, alcoxy en C₁-C₄, halogénoalcoxy en C₁-C₄ et alcoxyalcoxy en C₂-C₈; ou un reste -COR⁶, alkylsulfonyle en C₁-C₄, halogène, NO₂, -ER⁷, -NR⁸R⁹, ou -SO₂NR¹⁰R¹¹;
R² représente un atome d'hydrogène; un reste alkyle en C₁-C₃, non substitué ou substitué par alcoxy en C₁-C₃, hydroxy, alkylthio en C₁-C₂ ou CN; ou un reste halogénoalkyle en C₁-C₃, halogène, CN, NO₂, alcoxy en C₁-C₃, alkylthio en C₁-C₃, halogénoalcoxy en C₁-C₃, alkylamino en C₁-C₃, di(alkyl en C₁-C₃)amino ou alkylsulfonyle en C₁-C₃;
R³ est H ou CH₃;
R⁴, R⁵ représentent indépendamment l'un de l'autre un reste alkyle en C₁-C₃, alcoxy en C₁-C₃, halogénoalkyle en C₁-C₃, halogénoalcoxy en C₁-C₃, alkylthio en C₁-C₃, halogène, alcoxyalkyle en C₂-C₅ ou alkylamino en C₁-C₃;
X est CH ou N;
R⁶ représente un atome d'hydrogène ou un reste -NR¹⁰R¹¹, -ER¹² ou alkyle en C₁-C₄ non substitué ou substitué par un ou plusieurs restes du groupe constitué par des atomes d'halogène et des restes alcoxy en C₁-C₃ et alkylthio en C₁-C₃;
E est un atome d'oxygène ou de soufre;
R⁷ représente un reste alkyle en C₁-C₄ non substitué ou substitué par un ou plusieurs restes du groupe constitué par des atomes d'halogène et des restes alcoxy en C₁-C₃, alcényloxy en C₃-C₄, alcynyloxy en C₃-C₄, alkylthio en C₁-C₃ et (alcoxy en C₁-C₄)carbonyle; ou un reste alcényle en C₃-C₄ ou alcynyle en C₃-C₄;
R⁸ représente un reste alkyle en C₁-C₄, halogénoalkyle en C₁-C₄ ou alcoxyalkyle en C₂-C₆;
R⁹ représente un reste alkylsulfonyle en C₁-C₄, non substitué ou substitué par un ou plusieurs restes du groupe constitué par des atomes d'halogène et des restes alcoxy en C₁-C₄;
R¹⁰, R¹¹ représentent indépendamment l'un de l'autre un atome d'hydrogène ou un reste alkyle en C₁-C₃, halogénoalkyle en C₁-C₃, alcoxy en C₁-C₂, alcényle en C₃-C₄ ou alcynyle en C₃-C₄, ou
R¹⁰ et R¹¹ forment ensemble un pont alkylène de formule -(CH₂)ₙ- où n = 2, 3, 4 ou 5, le pont alkylène pouvant être interrompu par un atome d'oxygène; et
R¹² représente un reste alkyle en C₁-C₄ non substitué ou substitué par un ou plusieurs restes du groupe constitué par des atomes d'halogène et des restes alcoxy en C₁-C₃, alcényloxy en C₃-C₄, alcynyloxy en C₃-C₄, phénoxy, alkylthio en C₁-C₃ ou (alcoxy en C₁-C₄)carbonyle; ou un reste alcényle en C₃-C₄, alcynyle en C₃-C₄ ou phényle,
caractérisé en ce que l'on fait réagir une 1,1,3-trioxo-1,2,4-thiadiazolo[4,5-a]pyridine zwitterionique de formule (II) 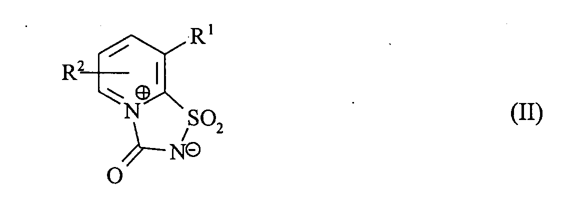 dans laquelle R¹ et R² ont la même signification que dans la formule (I),
avec un aminohétérocycle de formule (III) 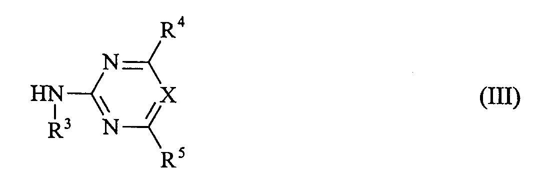 dans laquelle R³, R⁴, R⁵ et X ont la même signification que dans la formule (I), dans un solvant organique inerte.

2. Procédé selon la revendication 1, caractérisé en ce que
R¹ représente un reste alkyle en C₁-C₄ non substitué ou substitué par un ou plusieurs atomes d'halogène ou par un ou deux restes hydroxy ou alcoxy en C₁-C₄, ou par un ou plusieurs atomes d'halogène et un ou deux restes hydroxy ou alcoxy en C₁-C₄; ou un reste -COR⁶, alkylsulfonyle en C₁-C₄, halogène ou -NR⁸R⁹;
R² représente un atome d'hydrogène; un reste alkyle en C₁-C₃, non substitué ou substitué par alcoxy en C₁-C₃, hydroxy, alkylthio en C₁-C₂ ou CN; ou un reste alkylthio en C₁-C₃, halogénoalcoxy en C₁-C₃, alkylamino en C₁-C₃ ou di(alkyl en C₁-C₃)amino;
R³ est H ou CH₃;
R⁴, R⁵ représentent indépendamment l'un de l'autre un reste alkyle en C₁-C₂, alcoxy en C₁-C₃, halogénoalkyle en C₁-C₂, halogénoalcoxy en C₁-C₂, halogène ou alkylamino en C₁-C₂;
X est CH ou N;
R⁶ représente un atome d'hydrogène ou un reste -ER¹² ou alkyle en C₁-C₄;
E est un atome d'oxygène;
R⁸ représente un reste alkyle en C₁-C₄ ou halogénoalkyle en C₁-C₄;
R⁹ représente un reste alkylsulfonyle en C₁-C₄; et
R¹² représente un reste alkyle en C₁-C₄.

3. Procédé selon la revendication 1 ou 2, caractérisé en ce que l'on utilise un solvant organique inerte du groupe constitué par les hydrocarbures aromatiques, les hydrocarbures halogénés aliphatiques et aromatiques, les éthers, les esters, les cétones, les nitriles et les amides, et leurs mélanges.

4. Procédé selon la revendication 3, caractérisé en ce que l'on utilise le chlorobenzène ou le dichlorométhane comme solvant organique inerte.

5. Procédé selon la revendication 3 ou 4, caractérisé en ce que l'on effectue la réaction à des températures comprises entre -10°C et la température d'ébullition du solvant.

6. Procédé selon la revendication 5, caractérisé en ce que les températures sont comprises entre 0°C et 80°C.

7. Composés de formule II 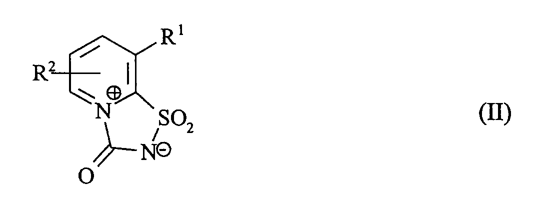 dans laquelle R¹ et R² ont la même signification que dans la formule (I) selon la revendication 1 ou 2.

8. Procédé de préparation de composés de formule II selon la revendication 7, caractérisé en ce que l'on fait réagir des composés de formule (IV) 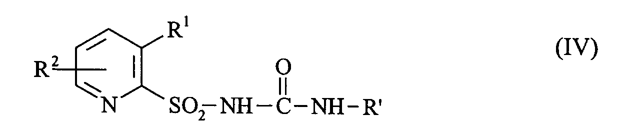 dans laquelle R¹ et R² ont la définition indiquée pour la formule (II) et R' représente un reste alkyle en C₁-C₈, avec du phosgène, en présence d'un solvant organique inerte, à des températures comprises entre 70°C et le point d'ébullition du solvant.

9. Procédé selon la revendication 8, caractérisé en ce que l'on effectue la réaction à des températures comprises entre 70°C et 140°C.

10. Procédé de préparation de composés de formule II selon la revendication 7, caractérisé en ce que l'on fait réagir un composé de formule V 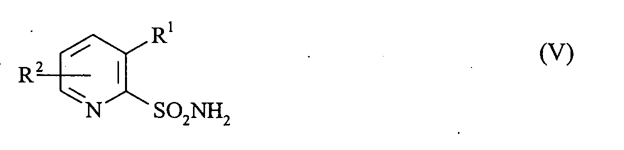 dans laquelle R¹ et R² ont la même définition que dans la formule (II) en présence d'un isocyanate d'alkyle en C₁-C₈ et de phosgène.

11. Procédé selon la revendication 10, caractérisé en ce que la réaction s'effectue dans un solvant organique inerte à des températures comprises entre 70°C et le point d'ébullition du solvant.

12. Procédé selon la revendication 8 ou 9 ou 10 ou 11, caractérisé en ce que, pour accélérer la réaction, on utilise un catalyseur du groupe des amines tertiaires.

13. Utilisation de composés de formule II selon la revendication 7 pour la préparation de sulfonylurées.
